# EUROPEAN PATENT APPLICATION

(11) **EP 4 249 546 A1**
(43) Date of publication of application: **27.09.2023**
(21) Application number: 23163575.6
(22) Date of filing: 22.03.2023
(51) Int. Cl.: C08J 9/14, C08G 2/18, C08G 61/12, C07B 41/02, C07C 49/00, C07C 67/31, C07C 67/30, C08G 59/02, C08G 59/14

(54) **PLANT OIL-BASED POLYMER FOAM AND METHOD OF MANUFACTURE THEREOF**

(30) Priority: 22.03.2022 WO PCT/IB2022/052590
(71) Applicant: LATVIJAS VALSTS KOKSNES KIMIJAS INSTITUTS, 1006 Riga (LV)
(72) Inventor: KIRPLUKS, Mikelis, 1006 Riga (LV); POMILOVSKIS, Ralfs, 1006 Riga (LV); FRIDRIHSONE, Anda, 1006 Riga (LV); ABOLINS, Arnis, 1006 Riga (LV)
(74) Representative: Kromanis, Artis

(57) **Abstract**

The invention relates to a method of manufacture of a thermoset polymer foam that is at least from 50 to 100 percent by weight of renewable raw material resources and to the foam obtained by the method. Foam is obtained from a two-component system that cures at room temperature after mixing in the presence of a strong base catalyst by carbon-Michael addition polymerization, forming mechanically rigid and highly cross-linked polymer. Both components of the carbon-Michael addition are developed from bio-based feedstock.

## Description

### FIELD OF THE INVENTION

The invention relates to a method of manufacture of a thermoset polymer foam that is at least from 50 to 100 percent by weight of renewable raw material resources. Foam is obtained from a two-component system that cures at room temperature after mixing in the presence of a strong base catalyst by carbon-Michael addition polymerization, forming mechanically rigid and highly cross-linked polymer. Both components of the carbon-Michael addition are developed from bio-based feedstock.

### BACKGROUND OF THE INVENTION

The main challenge of the polyurethane foam material industry is associated with the production and handling of isocyanates. It is unlikely that urethane chemistry will be displaced as it exhibits very high effectiveness and highly advantageous economies of scale. However, for instances where polyurethane foam chemistry is undesired, it is very beneficial to have a useful alternative.

EP1162222A2 discloses a low index polyurethane foam that is at least partially biodegradable, comprising the reaction product of from about 10 to about 35 percent by weight of an isocyanate; from about 90 to about 65 percent by weight of active hydrogencontaining component selected from the group consisting of from about 80 to about 100 percent by weight of a component derived from a natural, renewable component and from about 0 to about 20 percent by weight of a component derived from a petrochemical source to total 100 percent by weight of active hydrogen containing component; from about 0.5 to about 1 percent by weight of a catalyst from about 0.3 to about 3 percent by weight of a surfactant; and from about 0.5 to about 20 percent by weight of a blowing agent. The disclosure includes a component derived from a natural, renewable component, however, still uses from about 10 to about 35 percent by weight of an isocyanate.

PCT/US2019/018710 proposes methods of making isocyanate-free foam using isocyanate-free polyurethane chemistry. The disclosed formulation has the first part with at least one multifunctional acrylate. The disclosed formulations have a second part with at least one non-isocyanate polyurethane oligomer derived from a reaction of at least one multifunctional cyclocarbonate and at least one first multifunctional amine, and a second multifunctional amine. The formulation may also have a blowing agent and at least one surfactant. Although the method uses isocyanate-free polyurethane chemistry, it still requires feedstock that is not bio-based.
US20080281006A1 proposes a method of making one-part non-toxic spray foam by Michael addition chemistry. The disclosed formulation contains multifunctional acetoacetates and acrylates. However, acetoacetates and acrylates are not based on a bio-based feedstock such as bio-based polyols that are obtained from various bio-based fatty acids and fatty acid triglycerides.
EP 3 783 048 A1 proposes a method of applying a foam composition using spray foam equipment based on multi-functional acetoacetate esters and multi-functional amines or acrylates. The method describes use of diacrylate compounds, whereas this patent describe application of multifunctional bio-based acrylates. The acetoacetates are based on different compounds, mainly sugar derivatives, whereas the acetoacetates presented in this patent are based on bio-based polyols derived from bio-based fatty acids and fatty acid triglycerides.
US20060069234A1 discloses a polymer compositions comprising at least one multifunctional Michael acceptor, at least one multi-functional Michael donor, in which at least 20% of either the donor or acceptor or a combination of the donor and acceptor is derived from bio-based materials and at least one catalyst. The disclosure includes acceptor formation by reacting bio-based epoxide with acrylic acid, still bio-based polyol as a feedstock and acrylation with acryloyl chloride as acceptor synthesis method are not included. The disclosure includes foam however, still the formulation of foam including catalyst and/or surfactant and/or blowing agent and/or others is not defined.

### SUMMARY OF THE INVENTION

The objective of the invention is to provide sustainable method of production of polymer foam that would exploit only bio-based feedstock.

The goal is achieved by the invented method, which comprises the following steps:
a) providing a plant oil;
b) epoxidation of a double bond of the plant oil of step (a);
c) obtaining of an epoxidized plant oil polyol, that includes heating up the epoxidized plant oil at about 80 °C to 250 °C, preferably about 180 °C, for about 5 h to 10 h;
d) obtaining of Michael donor of Formula (II) and Michael acceptor of Formula (III): 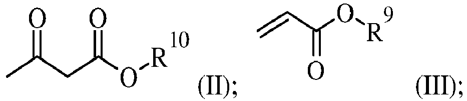
   wherein R¹⁰ is plant oil based moiety selected from the group comprising hydrogen, alkyl, alkenyl, oxirane group, hydroxyl -OH, ester -C(=O)-O-, -C=C-, -C=C-, carboxyl group -C(=O)-OH, -C-O-C-, -C=C, acetoacetate -O-C(=O)-C-C(=O)-C, β-ketoester group -O-C(=O)-C-C(=O)-C-, nitrogen-containing group;
   wherein R⁹ is plant oil based moiety selected from the group comprising hydrogen or organic radicals such as alkyl (linear, branched, or cyclic), alkenyl, oxirane group, hydroxyl -OH, ester -C(=O)-O-, -C=C-, -C=C-, carboxyl group C(=O)-OH, -C-O-C-, - C=C, acrylic -O-C(=O)-C=C, methacrylic -O-C(=O)-C(=C)-C, thio analogs thereof, nitrogen-containing groups, or combinations thereof, including derivatives and substituted versions thereof;
e) mixing of the Michael donor with the Michael acceptor obtained in step (d) in the presence of a catalyst, adding a surfactant and a blowing agent, resulting in a reaction mixture;
f) heating up the reaction mixture for foam forming up to a temperature from 0 °C to 80 °C, preferably from 25 °C to 55 °C;
g) mixing of the reaction mixture for 1 s to 300 s, preferably for 3 s to 120 s, more preferably for 5 s to 60 s;
in the result of steps e), f), and g) the blowing agent is expanded, forming the polymeric foam material. This method and its defined steps in correct order provides sustainable production of polymer foam that exploits bio-based feedstock. Especially the steps a)-c) allows to obtain epoxidized plant oil polyol that can be processed further to the polymeric foam material in less energy consuming way. Step a) providing a plant oil is a requirement for the use of plant oil to ensure that the resulting Michael donor and Michael acceptor components are bio-based; step b) epoxidation of a double bond of the plant oil of step (a) is a requirement for obtaining polyol precursors; step c) obtaining of an epoxidized plant oil polyol, that includes heating up the epoxidized plant oil at about 80 °C to 250 °C, preferably about 180 °C, for about 5 h to 10 h, provides oxirane opening reaction with alcohol to obtain the precursor for Michael donor and Michael acceptor components.

The examples of plant oil include, but are not limited to coconut oil; canola oil; castor oil; coconut oil; colza oil; copaiba oil; corn oil; cottonseed oil; false flax oil; hemp oil; jatropha oil; jojoba oil; milk bush oil; mustard oil; nahor oil; nut oils, such as almond oil, brazil nut oil, cashew oil, hazelnut oil, macadamia oil, pecan oil, pine nut oil, pistachio oil, walnut oil; linseed oil; olive oil; palm oil; pongamia oil; radish oil; ramtil oil; rapeseed oil; rice bran oil; safflower oil; salicornia oil; sesame oil; soybean oil; sunflower oil; tall oil and mixtures and derivatives thereof;

The plant oils used in the invention contain a mixture of one or more unsaturated fatty acids including, but not limited to arachidonic acid; cervonic acid; dihomo-γ-linolenic acid; docosatetraenoic acid; eicosapentaenoic acid; elaidic acid; erucic acid; gondoic acid; linoleic acid; linolelaidic acid; mead acid; nervonic acid; oleic acid; palmitoleic acid; paullinic acid; stearidonic acid; vaccenic acid; α-linolenic acid; γ-linolenic acid; ricinoleic acid.

The plant oil may contain monoglycerides, diglycerides and triglycerides. Monoglycerides are esters derived from glycerol and one fatty acid. Diglycerides are esters derived from glycerol and two fatty acids. Triglycerides are esters derived from glycerol and three fatty acids. Triglycerides and diglycerides may contain both saturated and unsaturated fatty acid groups. All reactions and transformations also apply to the functional groups contained in molecules of diglycerides and triglycerides.

The epoxidation is carried out by obtaining a mixture of the plant oil comprising a double bond and peroxyacid that transform a part or all of the oil's double bonds to oxirane groups, that can be added to the reaction mixture directly or created *in-situ* by reacting a hydroperoxide with a corresponding acid such as formic acid, acetic acid, trifluoroacetic acid, benzoic acid, benzyloxy formic acid, benzyloxyacetic acid, and heating 40 °C to 60 °C, then adding hydrogen peroxide while monitoring a temperature of the mixture so that it does not exceed 50 °C to 70 °C, preferably 60 °C, after the addition of hydrogen peroxide, the temperature is kept at 50 °C to 70 °C, preferably 60 °C for 4 h to 8 h, preferably 6 h in an inert environment provided by argon or nitrogen gas, the obtained mixture is washed with a distilled water having a temperature in a range of 40 °C to 80 °C, preferably about 60 °C, resulting in an epoxidized plant oil of Formula (I): 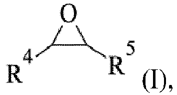 wherein
R⁴ and R⁵ are plant oil based moieties where each selected from hydrogen, alkyl, alkenyl, oxirane group, hydroxyl -OH, ester -C(=O)-O-, -C=C-, -C=C-, carboxyl group -C(=O)-OH, -C-O-C-, -C=C, nitrogen-containing group;

When the plant oil of step (a) is selected from a group, comprising coconut oil; canola oil; castor oil; coconut oil; colza oil; copaiba oil; corn oil; cottonseed oil; false flax oil; hemp oil; jatropha oil; jojoba oil; milk bush oil; mustard oil; nahor oil; linseed oil; olive oil; palm oil; pongamia oil; radish oil; ramtil oil; rapeseed oil; rice bran oil; safflower oil; salicornia oil; sesame oil; soybean oil; sunflower oil; tall oil; and mixtures and derivatives thereof, the epoxidized plant oil polyol is obtained by opening the oxirane ring of the epoxidized plant oil of step (b).

When the plant oil of step (a) is selected from a group, comprising tall oil and mixtures and derivatives thereof, the epoxidized plant oil polyol is obtained by oxirane ring-opening of the epoxidized plant oil of step (b), and subsequent carboxyl group esterification with alcohols. The oxirane ring-opening can take place both in the presence and absence of a ring-opening catalyst, wherein the ring-opening catalyst is selected from boronic acids; hydrobromic acid; hydrochloric acid; perchloric acid; perchloric acid metal salts, such as lithium perchlorate; phosphoric acid; phosphorous acid; sulfonic acids, such as para-toluene sulfonic acid, methanesulfonic acid, and trifluoromethane sulfonic acid; sulfuric acid; tetrafluoroboric acid; tetrafluoroboric acid metal salts, such as copper tetrafluoroborate and iron tetrafluoroborate; triflic acid and ion exchange resins in the protic form. The concentration of the ring-opening catalyst is in a range of 0.01 wt% to about 1 wt%, preferably from 0.05 wt% to 0.5 wt%. The alcohol is selected from oxirane ring-opening alcohols, comprising 2-ethyl-1-hexanol; 2-methyl-2-butanol; ethanol; isobutanol; isopropanol; methanol; n-butanol; n-propanol; propanol; 1,2-cyclohexanediol; 1,3-butanediol; 1,3-propanediol; 1,4-butanediol; 1,5-pentanediol; 1,6-hexanediol; 1,12-dodecanediol; 2,3-butanediol; 2-methyl-1,3-propandiol; 3-methyl-1,5-pentanediol; diethanolamine; ethylene glycol; hexanediol; pentanediol; propylene glycol; triethylene glycol; tripropylene glycol; erythritol; glycerol; pentaerythritol; triethanolamine; trimethylolethane; trimethylolpropane and mixtures thereof.

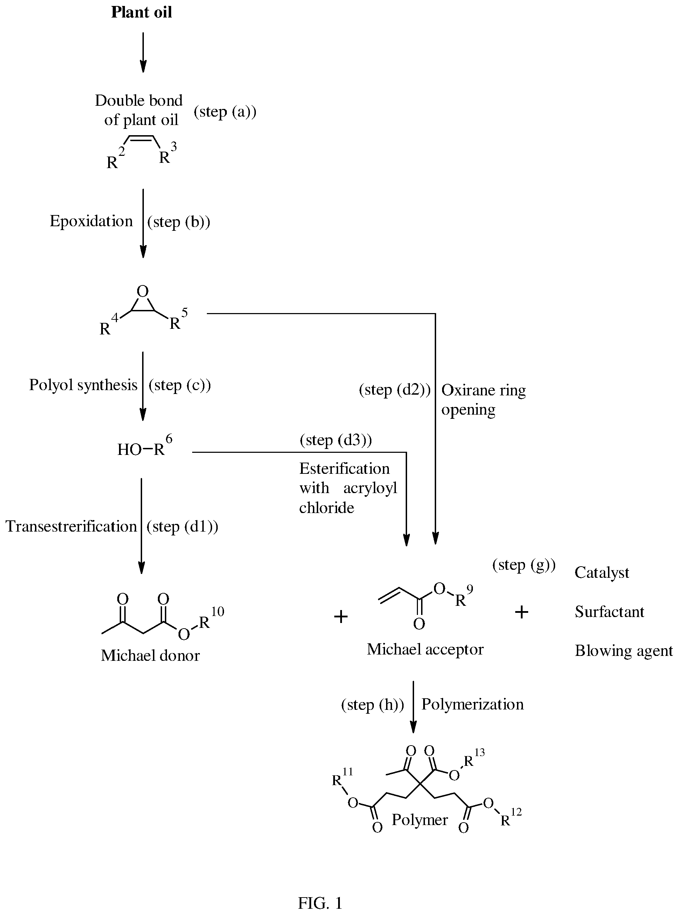

The Michael donor is obtained by subjecting the epoxidized plant oil polyol of step (c) to transesterification (see step (d1) in Fig. 1). The transesterification is performed using an acetoacetylating reagent at a temperature in a range of 100 °C to 140 °C, preferably about 120 °C, for 3 h to 8 h, preferably 4 h.

When the plant oil of step (a) is selected from a group, comprising coconut oil; canola oil; castor oil; coconut oil; colza oil; copaiba oil; corn oil; cottonseed oil; false flax oil; hemp oil; jatropha oil; jojoba oil; milk bush oil; mustard oil; nahor oil; linseed oil; olive oil; palm oil; pongamia oil; radish oil; ramtil oil; rapeseed oil; rice bran oil; safflower oil; salicornia oil; sesame oil; soybean oil; sunflower oil; tall oil and mixtures and derivatives thereof, the Michael acceptor is obtained by opening an oxirane ring of the epoxidized plant oil of step (b) by acrylating the epoxidized plant oil of step (b) by acrylic group containing acid or an anhydride at a temperature in range from 60 °C to 130 °C, preferably from 100 °C to 110 °C for 1 h to 24 h, then washing with a distilled water having a temperature in a range of 40 °C to 80 °C, and drying (see step (d2) in Fig. 1). The opening of the oxirane ring can take place both in the presence and absence of the free radical inhibitors. Examples of suitable free radical inhibitors include but are not limited to substances based on phenolic groups such as hydroquinones; hydroquinone; catechols; phenothiazines; phenonthiazine and mixtures thereof. Examples of suitable acid for acrylation include but are not limited to acrylic acid; methacrylic acid and mixtures thereof. Examples of suitable anhydride for acrylation include but are not limited to acrylic anhydride; methacrylic anhydride and mixtures thereof.

When the plant oil of step (a) is selected from a group, comprising tall oil and mixtures and derivatives thereof, the Michael acceptor is obtained by acrylation of the epoxidized plant oil polyol of step (c) with acryloyl chloride or methacryloyl chloride in triethanolamine and ethyl acetate solution at a temperature in a range from -5 °C to 5 °C and then stirring the resulting mixture at a temperature in a range from 10 °C to 30 °C for 12 h to 24 h (see step (d3) in Fig. 1). Acrylation can take place both in the presence and absence of free radical inhibitors. Examples of suitable free radical inhibitors include but are not limited to substances based on phenolic groups such as hydroquinones; hydroquinone; catechols; phenothiazines; phenonthiazine and mixtures thereof.

A ratio of the Michael acceptor functional groups to the Michael donor functional groups in step (d) is in a range of 1:1 to 3:1, preferably 1.5:1 to 2.5:1, more preferably 1.9:1 to 2.1:1.

The concentration of the catalyst is in a range of 0.05 wt% to 10 wt% based upon the total weight of the Michael donor and the Michael acceptor.

The catalyst is selected from the group comprising aliphatic amines; alkanolamines; aromatic amines; amidine; imine; imidazoles; metal salts; nitrogen; 1-(dmethylamino)-2-propanol; 1,1,3,3-tetramethylguanidine; 1,2-dimethylimidazole; 1,4-diazabicyclo[2.2.2]octane; 1,5,7-triazabicyclo[4.4.0]dec-5-ene; 1,5-diazabicyclo[4.3.0]non-5-ene; 1,8-bis(tetramethylguanidino)naphthalene; 1,8-diazabicyclo[5.4.0]undec-7-ene; 1-[bis[3-(dimethylamino)propyl]amino]-2-propanol; 1-[N,N-bis(2-hydroxyethyl)amino]-2-propanol; 1-azabicyclo[3.3.0]octane; 1-ethylpiperidine; 2-((dimethylamino)ethyl)methylaminopropanol; 2-(diethylamino)ethanol; 2-(diisopropylamino)ethanol; 2-(dimethylamino)ethyl methacrylate; 2-[2-(dimethylamino)ethoxy]ethanol; 2-ethyl-4-methyl-1H-imiazole; 2-tert-butyl-1,1,3,3-tetramethylguanidine; 4-butanediamine; 7-methyl-1,5,7-triazabicyclo[4,4,0]dec-5-ene; bis(3-aminopropyl)amine; bis(N,N-dimethyl-3-aminopropyl)amine; N-(2-aminopropyl)imidazole; N-(2-hydroxypropyl)-2-methylprop-2-enamide; N-(2-hydroxypropyl)imidazole; N-(3-aminopropyl)-2-pyrrolidinone; N,N,N',N'-tetramethylhexanediamine; N,N,N',N'-tetraacetylethylenediamine; N,N,N',N'-tetramethyl-1,3-propanediamine; N,N,N',N'-tetramethy1-1,4-butanediamine; N,N,N',N'-tetramethyl-1,6-hexanediamine; N,N,N',N'-tetramethyl-2-butene-1,4-diamine; N,N,N',N'-tetramethyldiaminomethane; N,N,N'-trimethyl-N'-hydroxyethyl bis(aminoethyl) ether; N,N-bis(3-dimethylaminopropyl)N-isopropanolamine; N,N-dimefhylpiperazine; N,N-dimethylaminopropylamine; N,N-dimethylaminopropyl-N'-methylethanolamine; N,N-dimethylefhanolamine; N,N-dimethyl-N'-ethylethylenediamine; N,N-dimorpholinodiethylether; N'-butyl-N',N'-dicyclohexylguanidine; N-ethylmorpholine; N-methylimidazole; N-methylmorpholine; potassium hydroxide; potassium phenoxide; potassium phosphate; sodium hydroxide; sodium phenoxide; tertraethylammonium hydroxide; tetramethylamino bis (propylamine); tetramethylammonium hydroxide; tetramethylguanidine; triazabcyclodecene; triethanolamine; trimethylamine; triethylenediamine; trimethylamine; tris(dimethylamino propyl)amine; tris(dimethylaminopropyl)amine; tris-isopropanolamine; N,N,N',N'-tetramethyl-1,4-butanediamine; N,N,N',N'-tetramethylguanidine; N,N-dimethylaminopropyldipropanolamine; N,N-dimethylbenzylamine; N,N-dimethylethanolamine; N,N-dimorpholinodiethyl ether and mixtures thereof, preferably nitrogen.

The concentration of the surfactant is in a range of 0.1 wt% to 10 wt% based upon the total weight of the Michael donor and the Michael acceptor.

Suitable surfactants that can be used in the invention include but are not limited to alkoxylated polysiloxanes;ethoxylated fatty acids; salts of fatty acids; ethoxylated fatty alcohols; salts of sulfonated fatty alcohols; fatty acid esters of sorbitan; and fatty acid ester sorbitan ethoxylates; silicone glycol copolymer and mixtures thereof.

The concentration of the blowing agent is in a range of 0.5 wt% to 50 wt% based upon the total weight of the Michael donor and the Michael acceptor.

The blowing agents that can be used in the invention include compounds that have a boiling temperature in a range from -50 °C to +100 °C. A preferred boiling temperature for blowing agents is in a range from 0 °C to 50 °C, and a more preferred boiling temperature is in a range from 10 °C to 40 °C. The blowing agents expand during the exothermic polymerization reaction. The blowing agents that can be used in the invention include, but are not limited to hydrocarbons (cyclopentane; isopentane; n-pentane); hydrofluorocarbons; hydrochlorofluorocarbons; hydrofluorocarbons; chlorofluorocarbons; fluorocarbons; chlorocarbons; esters; aliphatic alcohols; carbon dioxide; air; argon; nitrogen and mixtures thereof. In some embodiments, the suitable blowing agent can be a physical blowing agent. Examples of blowing agents include, but are not limited to 1,1-difluoroethane; 1,1,1,2,3,4,4,5,5,5-decafluoropentane; 1,1,1,3,3-pentafluorobutane; 1,1,1,3,3-pentafluoropropane; 1,1,2,2,3-pentafluoropropane; 1,1,2,2-tetrafluoroethane; 1,1,4,4,4-hexafluoro-2-butene; 1,1-dichloro-1-fluoroethane; 1,2-dichloro-1, 1,2,2-tetrafluoroethane; 1,2-dichloropropane; 1,2-dichlorotetrafluoroethane; 1-chloro-1,1-difluoroethane; 2,2-dichloro-1,1,1-trifluoroethane; cis-1,1,1,4,4,4-hexafluoro-2-butene; cyclopentane; formaldehyde dimethylacetal; isobutane; isopentane; methylbutane; methylformate; n-butane; n-pentane; pentane; perfluoromethane; propane; trichloromonofluoromethane; trichloromonofluoromethane; trichlorotrifluoroethane; argon; carbon dioxide; nitrogen; and mixtures thereof.

One or more additives can be used in foam-forming compositions. Examples of additives that can be used in the invention include, but are not limited to, antioxidants; antistatic agents; biocides; colorants; cure promoters; dyes; fillers; flame retardants; foam cell nucleators; foaming agents; functionalized fillers; odor inhibitors; odoriferous substances; perfumes; pigment dispersing agents; plasticizers; preservatives; reactive fillers; UV stabilizers and mixtures thereof. The one or more additives is/are in a range of 0.10 wt% to 20.0 wt% based upon the total weight of the Michael donor and the Michael acceptor.

The peroxyacid for epoxidation is selected from the group comprising peroxyformic acid; peroxyacetic acid; trifluoroperoxyacetic acid; benzyloxyperoxy formic acid and mixtures thereof.

The acetoacetylating reagent is selected from the group comprising tert-butyl acetoacetate; methyl acetoacetate; ethyl acetoacetate; or isopropyl acetoacetate and mixtures thereof.

The carbon-Michael donor may react difunctionally with the carbon-Michael acceptor; therefore, this ratio should be no more than 3 moles of Michael acceptor functional groups per mole of carbon Michael donor functional groups. The ratio of Michael acceptor functional groups to Michael donor functional groups can be in a range from 1:1 to 3:1. The ratio Michael acceptor functional groups : Michael donor functional groups can be 1.1:1, 1.2:1, 1.3:1, 1.4:1, 1.5:1, 1.6:1, 1.7:1, 1.8:1, 1.9:1, 2:1, 2.1:1, 2.2:1, 2.3:1, 2.4:1, 2.5:1, 1.6:1, 2.7:1, 2.8:1, 2.9:1, 3:1. A more preferred ratio is from 1.5:1 to 2.5:1, and a more preferred ratio is from 1.9:1 to 2.1:1.

The catalyst can have a concentration of 0.05 wt% to 10 wt%based upon the total weight of the Michael donor and the Michael acceptor. The reaction mixture may contain 0.06 wt%, 0.07 wt%, 0.08 wt%, 0.09 wt%, 0.1 wt%, 0.2 wt%, 0.3 wt%, 0.4 wt%, 0.5 wt%, 0.75 wt%, 0.9 wt%, 1 wt%, 1.1 wt%, 1.25 wt%, 1.5 wt%, 1.75 wt%, 2 wt%, 2.5 wt%, 3 wt%, 3.5 wt%, 4 wt%, 5 wt%, 6 wt%, 7 wt%, 8 wt%, 9 wt%, 10 wt% catalyst, or any range between these values.

The surfactant can have a concentration of 0.1 wt%to 10 wt% based upon total weight of the Michael donor and the Michael acceptor. The reaction mixture for foam may contain 0.10 wt%, 0.15 wt%, 0.2 wt%, 0.25 wt%, 0.3 wt%, 0.4 wt%, 0.5 wt%, 0.6 wt%, 0.7 wt%, 0.8 wt%, 0.9 wt%, 1.0 wt%, 1.1 wt%, 1.2 wt%, 1.3 wt%, 1.4 wt%, 1.5 wt%, 1.75 wt%, 2 wt%, 2.5 wt%, 3 wt%, 3.5 wt%, 4 wt%, 4.5 wt%, 5 wt%, 5.5 wt%, 6 wt%, 6.5 wt%, 7 wt%, 7.5 wt%, 8 wt%, 8.5 wt%, 9 wt%, 9.5 wt%, 10 wt% surfactant, or any range between these values.

The blowing agent can have a concentration of 0.5 wt% to 50 wt% based upon the total weight of the Michael donor and the Michael acceptor. The reaction mixture for foam may contain 0.5 wt%, 0.55 wt%, 0.6 wt%, 0.65 wt%, 0.7 wt%, 0.75 wt%, 0.8 wt%, 0.85 wt%, 0.9 wt%, 0.95 wt%, 1 wt%, 1.1 wt%, 1.2 wt%, 1.3 wt%, 1.4 wt%, 1.5 wt%, 1.75 wt%, 2 wt%, 2.25 wt%, 2.5 wt%, 2.75 wt%, 3 wt%, 3.25 wt%, 3.5 wt%, 3.75 wt%, 4 wt%, 4.25 wt%, 4.5 wt%, 4.75 wt%, 5 wt%, 5.5 wt%, 6 wt%, 6.5 wt%, 7 wt%, 7.5 wt%, 8 wt%, 8.5 wt%, 9 wt%, 9.5 wt%, 10 wt%, 11 wt%, 12 wt%, 13 wt%, 14 wt%, 15 wt%, 16 wt%, 18 wt%, 20 wt%, 22 wt%, 24 wt%, 26 wt%, 28 wt%, 30 wt%, 32 wt%, 34 wt%, 36 wt%, 38 wt%, 40 wt%, 42 wt%, 44 wt%, 46 wt%, 48 wt%, 50 wt% blowing agent or any range between these values.

One or more additives can be used in foam forming compositions and mixtures thereof. The reaction mixture for foam may contain 0.1 wt%, 0.15 wt%, 0.2 wt%, 0.25 wt%, 0.3 wt%, 0.4 wt%, 0.5 wt%, 0.6 wt%, 0.7 wt%, 0.8 wt%, 0.9 wt%, 1.0 wt%, 1.1 wt%, 1.2 wt%, 1.3 wt%, 1.4 wt%, 1.5 wt%, 1.75 wt%, 2 wt%, 2.5 wt%, 3 wt%, 3.5 wt%, 4 wt%, 4.5 wt%, 5 wt%, 5.5 wt%, 6 wt%, 6.5 wt%, 7 wt%, 7.5 wt%, 8 wt%, 8.5 wt%, 9 wt%, 9.5 wt%, 10 wt%, 11 wt%, 12 wt%, 13 wt%, 14 wt%, 15 wt%, 16 wt%, 18 wt%, 20 wt% additives, or any range between these values.

The reaction mixture for foam forming may be heated to a temperature from 0 °C to 80 °C. The reaction mixture for foam forming may be heated to a more preferred temperature of 25 °C to 55 °C. The reaction mixture for foam forming may be heated to 25 °C, 26 °C, 27 °C, 28 °C, 29 °C, 30 °C, 31 °C, 32 °C, 33 °C, 34 °C, 35 °C, 36 °C, 37 °C, 38 °C, 39 °C, 40 °C, 41 °C, 42 °C, 43 °C, 44 °C, 45 °C, 46 °C, 47 °C, 48 °C, 49 °C, 50 °C, 51 °C, 52 °C, 53 °C, 54 °C, 55 °C or any range between these values. Preheated to the above temperatures before adding to and creating the reaction mixture may also be any individual component or a mixture of components in any possible variation.

Examples of mixture that can be preheated to the above temperatures before adding to and creating the reaction mixture include but are not limited to a mixture of Michael acceptor and catalyst, a mixture of Michael acceptor and catalyst and surfactant, a mixture of Michael acceptor and catalyst and surfactant and blowing agent, a mixture of Michael acceptor and catalyst and blowing agent, a mixture of Michael acceptor and catalyst, a mixture of Michael donor and catalyst and surfactant, a mixture of Michael donor and catalyst and surfactant and blowing agent, a mixture of Michael donor and catalyst and blowing agent, a mixture of catalyst and surfactant and blowing agent and others.

After adding components or during the addition of components to the reaction mixture, the reaction mixture is mixed and stirred using any suitable mixing and stirring equipment, including static mixing and stirring equipment, impingement mixing and stirring equipment, or other suitable mixing equipment.

The mixing time for a mixture containing a Michael donor and Michael acceptor may be from 1 s to 300 s. The more preferred mixing time for a mixture containing Michael donor and Michael acceptor may be 3 s to 120 s, and still more preferred mixing time for a mixture containing Michael donor and Michael acceptor may be 5 s to 60 s. The mixing time for a mixture containing a Michael donor and Michael acceptor may be 5 s, 6 s, 7 s, 8 s, 9 s, 10 s, 11 s, 12 s, 13 s, 14 s, 15 s, 16 s, 17 s, 18 s, 19 s, 20 s, 21 s, 22 s, 23 s, 24 s, 25 s, 26 s, 27 s, 28 s, 29 s, 30 s, 32 s, 34 s, 36 s, 38 s, 40 s, 42 s, 44 s, 46 s, 48 s, 50 s, 52 s, 54 s, 56 s, 58 s, 60 s or any range between these values.

Premixed may also be any individual component or a mixture of components in any possible variation.

The bio-based foam of the invention is a reaction product of the reaction of the mixture described above.

These blowing agents expand during the exothermic reaction of Michael donor and Michael acceptor to generate the blowing gas, which forms a foam structure. These formed foams are the bio-based foams of the invention.

Another aspect of the invention is the bio-based foams obtained by the method of the invention.

### BRIEF DESCRIPTION OF THE DRAWINGS

Figure 1 shows a principal scheme of manufacturing technology of a polymer.

### DETAILED DESCRIPTION OF THE INVENTION

The invention is described but not limited by the following examples:

### Example 1

| | |
|---|---|
| (b) Epoxidized rapeseed oil | Rapeseed oil (800 g), acetic acid (104 g) and ion exchange resin Amberlite IR 120 H (20 wt% of rapeseed oil, 160 g) was heated to 40 °C in a four-necked round flask (2 L) which was immersed in a thermostatic water bath. Then slowly and evenly, the hydrogen peroxide (496 g) was added through the dropping funnel while watching the temperature so that it did not exceed 60 °C. After the addition of hydrogen peroxide, the temperature was kept at 60 °C for 6 h, and the medium was stirred at 600 rpm. An inert environment was provided by argon gas. The obtained mixture was washed with warm distilled water (60 °C) and ethyl acetate and dried using a rotatory vacuum evaporator to separate epoxidized rapeseed oil. |
| (c) Epoxidized rapeseed oil trimethylolpropane polyol | The epoxidized rapeseed oil (300 g) was added via a dropping funnel to a four-neck flask (1 L) already containing the mixture of trimethylolpropane (148 g) (amount of epoxidized rapeseed oil oxirane group to trimethylolpropane moles ratio was 1:1) and tetrafluoroboric acid solution (48 wt% in H₂O, 0.74 g) as a catalyst. Flask was immersed in a thermostatic water bath. The temperature was maintained at 80 °C during the addition of the oil. In this step, the oxirane ring was opened. After all the epoxidized oil had been added, the temperature was raised to 180 °C. The stirring rate was 500 rpm. The duration of the reaction was 6 h from the beginning of the addition of epoxidized rapeseed oil. |
| (d1) Epoxidized rapeseed oil trimethylolpropane polyol acetoacetate | Via dropping funnel tert-butyl acetoacetate (219.8 g) was added to epoxidized rapeseed oil polyol (200 g) in the three-neck round bottom flask. Flask was immersed in a thermostatic water bath, and the temperature was kept constant at about 120 °C throughout the synthesis. Tert-butanol released during the reaction was condensed using a Liebig condenser using water as a cooling agent 20 °C. In total, the reaction was performed for 4 h to maximize conversions. As a result of this step, a Michael donor was obtained. |
| (d2) Epoxidized rapeseed oil acrylate | Via dropping funnel tert-butyl acetoacetate (219.8 g) was added to epoxidized rapeseed oil polyol (200 g) in the three-neck round bottom flask. Flask was immersed in a thermostatic water bath, and the temperature was kept constant at |
| | about 120 °C throughout the synthesis. Tert-butanol released during the reaction was condensed using a Liebig condenser using water as a cooling agent 20 °C. In total, the reaction was performed for 4 h to maximize conversions. |
| | The acrylic acid (82 g) was added via a dropping funnel to a four-neck flask (1 L) already containing the mixture of epoxidized rapeseed oil (300 g) and tetrafluoroboric acid solution (48 wt.% in H₂O, 0.9 g) as a catalyst. Flask was immersed in a thermostatic oil bath. The temperature was maintained at 105 °C during the addition of the oil. In this step, the oxirane ring was opened. The stirring rate was 500 rpm. The duration of the reaction was 4 h. As a result of this step a Michael acceptor was obtained. |
| (g)(h) Polymer foam | The mixture of epoxidized rapeseed oil acrylate (72 g), blowing agent 1,1,3,3 tetramethylguanidine (2 g), surfactant L-6918 (1 g), and blowing agent Solkane 87/15 (40 g) was premixed 10 s. After premixing, epoxidized rapessed oil trimethylolpropane polyol acetoacetate (23 g) was added to the mixture of epoxidized rapeseed oil acrylate, blowing agent 1,1,3,3 tetramethylguanidine, surfactant L-6918, and blowing agent Solkane 87/15. The mixture was mixed with 2000 rpm. The mixing time for a mixture containing a Michael donor, a Michael acceptor, a catalyst, and a blowing agent was 15 s. |
| | The blowing agents expand during the exothermic reaction of Michael donor and Michael acceptor to generate the blowing gas, which forms a foam structure in the presence of a surfactant. These formed foams are the bio-based foams of the invention. |
| | Characterization: |
| | Density ρ=0.14 g/cm3 |
| | The coefficient of thermal conductivity λ=34.8 W/(m·K) |

### Example 2

| | |
|---|---|
| (b) Epoxidized tall oil | Tall oil fatty acids (700 g), acetic acid (126 g) and ion exchange resin Amberlite IR 120 H (20 wt% of tall oil, 140 g) was heated to 40 °C in a four-necked round flask (2 L) which was immersed in a thermostatic water bath. Then slowly and evenly, the hydrogen peroxide (614 g) was added through the dropping funnel at the same time watching the temperature so that it does not exceed 60 °C. After the addition of hydrogen peroxide, the temperature was kept at 60 °C for 6 h, and the medium was stirred at 600 rpm. An inert environment was provided by argon gas. The obtained mixture was washed with warm distilled water (60 °C) and ethyl acetate and dried using a rotatory vacuum evaporator to separate epoxidized tall oil. |
| (c) Epoxidized tall oil trimethylolpropane polyol | The epoxidized tall oil (300 g) was added via a dropping funnel to a four-neck flask (1 L) already containing the mixture of trimethylolpropane (200 g) (amount of epoxidized tall oil fatty acids carboxyl and oxirane group to trimethylolpropane moles ratio was 1:1) and tetrafluoroboric acid solution (48 wt.% in H₂O, 0.9 g) as a catalyst. Flask was immersed in a thermostatic water bath. The temperature was maintained at 80 °C during the addition of the oil. In this step, the oxirane ring was opened. After all the epoxidized oil had been added, the temperature was raised to 180 °C. The stirring rate was 500 rpm. The water formed during the synthesis was removed with an inert carrier gas (nitrogen) and condensed in the Liebig condenser. The duration of esterification was 6 h from the beginning of the addition of epoxidized tall oil. |
| (d1) Epoxidized tall oil trimethylolpropane polyol acetoacetate | Via dropping funnel tert-butyl acetoacetate (175.6 g) was added to epoxidized tall oil fatty acids trimethylolpropane polyol (150.0 g) in the three-neck round bottom flask (1 L). Flask was immersed in a thermostatic oil bath, and the temperature was kept constant at about 120 °C throughout the synthesis. |
| | Released tert-butanol during the reaction was condensed using a Liebig condenser using water as a cooling agent 20 °C. In total, the reaction was performed for 4 h to maximize conversions. As a result of this step, a Michael donor was obtained. |
| (d3) Epoxidized tall oil trimethylolpropane polyol acrylate | Epoxidized tall oil fatty acid trimethylolpropane polyol (50 g), triethylamine (62.5 g) and ethyl acetate (250 g) were chilled in an ice bath after being put in a three-necked flask (1 L) with a magnetic stirrer and a reflux condenser. Under an inert atmosphere of nitrogen, acryloyl chloride (37 g) that contains -400 ppm phenothiazine as an inhibitor (stabilizer) was added dropwise to the mixture. |
| | After the addition, the reaction temperature was maintained in a range between 0 to 5 °C for 4 h. At room temperature, the reaction was continued for another 18 h. The inorganic precipitate was then filtered off, and the organic phase was washed multiple times with a 1 % sodium hydroxide solution and distilled water, followed by an overnight drying with anhydrous sodium sulphate. Yellow oil was recovered when the solvent was evaporated using a rotary evaporator under decreased pressure. As a result of this step, a Michael acceptor was obtained. |
| (g)(h) Polymer foam | The mixture of epoxidized tall oil fatty acids trimethylolpropane polyol acrylate (72.1 g), blowing agent 1,1,3,3-tetramethylguanidine (2 g), surfactant L-6918 (1 g), and blowing agent Solkane 87/15 (40 g) was premixed 10 s. After premixing, epoxidized tall oil fatty acids trimethylolpropane polyol acetoatetate (27.9 g) was added to the mixture of epoxidized tall oil fatty acids trimethylolpropane polyol acrylate, blowing agent 1,1,3,3 tetramethylguanidine, surfactant L-6918, and blowing agent Solkane 87/15. The mixture was mixed with 2000 rpm. The mixing time for a mixture containing a Michael donor, a Michael acceptor, a catalyst and a blowing agent was 15 s. |
| | The blowing agents expand during the exothermic reaction of Michael donor and Michael acceptor to generate the blowing gas, which forms a foam structure in the presence of a surfactant. These formed foams are the bio-based foams of the invention. |
| | Characterization: |
| | Density ρ=0.11 g/cm³ |
| | The coefficient of thermal conductivity λ=34.5 mW/(m·K) |

### Example 3

| | |
|---|---|
| (b) Epoxidized tall oil | Tall oil fatty acids (700 g), acetic acid (126 g) and ion exchange resin Amberlite IR 120 H (20 wt% of TOFA, 140 g) were heated to 40 °C in a four-necked round flask (2 L) which was immersed in a thermostatic water bath. Then slowly and evenly the hydrogen peroxide (614 g) was added through the dropping funnel at the same time watching the temperature so that it does not exceed 60 °C. After |
| | the addition of hydrogen peroxide, the temperature was kept at 60 °C for 6 h, and the medium was stirred at 600 rpm. An inert environment was provided by argon gas. The obtained mixture was washed with warm distilled water (60 °C) and ethyl acetate and dried using a rotatory vacuum evaporator to separate epoxidized tall oil fatty acids. |
| (b') Epoxidized rapeseed oil | Rapeseed oil (800 g), acetic acid (104 g) and ion exchange resin Amberlite IR 120 H (20 wt% of rapeseed oil, 160 g) was heated to 40 °C in a four-necked round flask (2 L) which was immersed in a thermostatic water bath. Then slowly and evenly, the hydrogen peroxide (496 g) was added through the dropping funnel at the same time watching the temperature so that it does not exceed 60 °C. After the addition of hydrogen peroxide, the temperature was kept at 60 °C for 6 h, and the medium was stirred at 600 rpm. An inert environment was provided by argon gas. The obtained mixture was washed with warm distilled water (60 °C) and ethyl acetate and dried using a rotatory vacuum evaporator to separate epoxidized rapeseed oil. |
| (c) Epoxidized tall oil trimethylolpropane polyol | Epoxidized tall oil (300 g) was added via a dropping funnel to a four-neck flask (1 L) already containing the mixture of trimethylolpropane (200 g) (amount of epoxidized tall oil fatty acids carboxyl and oxirane group to trimethylolpropane moles ratio was1:1) and tetrafluoroboric acid solution (48 wt% in H₂O, 0.9 g) as a catalyst. Flask was immersed in a thermostatic water bath. The temperature was maintained at 80 °C during the addition of the oil. In this step, the oxirane ring was opened. After all the epoxidized oil had been added, the temperature was raised to 180 °C. The stirring rate was 500 rpm. The water formed during the synthesis was removed with an inert carrier gas (nitrogen) and condensed in the Liebig condenser. The duration of esterification was 6 h from the beginning of the addition of epoxidized tall oil. |
| (d1) Epoxidized tall oil trimethylolpropane polyol acetoacetate | Via dropping funnel tert-butyl acetoacetate (175.6 g) was added to epoxidized tall oil fatty acid polyol (150.0) in the three-neck round bottom flask. Flask was immersed in a thermostatic water bath, and the temperature was kept constant at about 120 °C throughout the synthesis. Tert-butanol released during the reaction was condensed using a Liebig condenser using water as a cooling agent 20 °C. |
| | In total, the reaction was performed for 4 h to maximize conversions. As a result of this step, a Michael donor was obtained. |
| (d2) Epoxidized rapeseed oil acrylate | The acrylic acid (82 g) was added via a dropping funnel to a four-neck flask (1 L) already containing the mixture of epoxidized rapeseed oil (300 g) and tetrafluoroboric acid solution (48 wt.% in H₂O, 0.9 g) as a catalyst. Flask was immersed in a thermostatic oil bath. The temperature was maintained at 105 °C during the addition of the oil. In this step, the oxirane ring was opened. The stirring rate was 500 rpm. The duration of the reaction was 4 h. As a result of this step, a Michael acceptor was obtained. |
| (g)(h) Polymer foam | The mixture of epoxidized rapeseed oil acrylate (72.1 g) (Michael acceptor), blowing agent 1,1,3,3-tetramethylguanidine (2 g), surfactant L-6918 (1 g), and blowing agent Solkane 87/15 (40 g) was premixed 10 s. After premixing, epoxidized tall oil trimethylolpropane polyol acetoacetate (23 g) (Michael donor) was added to the mixture of epoxidized rapeseed oil acrylate, blowing agent 1,1,3,3 tetramethylguanidine, surfactant L-6918, and blowing agent Solkane 87/15. The mixture was mixed with 2000 rpm. The mixing time for a mixture containing a Michael donor, a Michael acceptor, a catalyst and a blowing agent was 15 s. |
| | The blowing agents expand during the exothermic reaction of Michael donor and Michael acceptor to generate the blowing gas, which forms a foam structure in |
| | the presence of a surfactant. These formed foams are the bio-based foams of the invention. |
| | Characterization: |
| | Density ρ=0.13 g/cm³ |
| | The coefficient of thermal conductivity λ=35.5 mW/(m·K) |

## Claims

1. A method of manufacture of polymeric foam material, wherein the method comprises the following steps:
a) providing a plant oil, wherein the plant oil is selected from a group, comprising coconut oil; canola oil; castor oil; coconut oil; colza oil; copaiba oil; corn oil; cottonseed oil; false flax oil; hemp oil; jatropha oil; jojoba oil; milk bush oil; mustard oil; nahor oil; linseed oil; olive oil; palm oil; pongamia oil; radish oil; ramtil oil; rapeseed oil; rice bran oil; safflower oil; salicornia oil; sesame oil; soybean oil; sunflower oil; tall oil and mixtures and derivatives thereof;
b) epoxidation of a double bond of the plant oil of step (a),
wherein the epoxidation comprises obtaining a mixture of the plant oil comprising a double bond and peroxyacid that transform a part or all of the oil's double bonds to oxirane groups, that can be added to the reaction mixture directly or created *in-situ* by reacting a hydroperoxide with a corresponding acid such as formic acid, acetic acid, trifluoroacetic acid, benzoic acid, benzyloxy formic acid, benzyloxyacetic acid, and heating to 40-60 °C, then adding hydrogen peroxide while monitoring a temperature of the mixture so that it does not exceed 50-70 °C, preferably 60 °C, after the addition of hydrogen peroxide, the temperature is kept at 50-70 °C, preferably 60 °C for 4 to 8 hours, preferably 6 hours in an inert environment provided by argon or nitrogen gas, the obtained mixture is washed with a distilled water having a temperature in a range of 40 to 80 °C, preferably about 60 °C, resulting in an epoxidized plant oil of Formula (I): 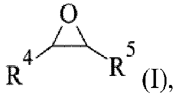 wherein
R⁴ and R⁵ are plant oil based moieties where each selected from hydrogen, alkyl, alkenyl, oxirane group, hydroxyl -OH, ester -C(=O)-O-, -C=C-, -C=C-, carboxyl group -C(=O)-OH, -C-O-C-, -C=C, nitrogen-containing group;
c) obtaining of an epoxidized plant oil polyol:
wherein, when the plant oil of step (a) is selected from a group, comprising coconut oil; canola oil; castor oil; coconut oil; colza oil; copaiba oil; corn oil; cottonseed oil; false flax oil; hemp oil; jatropha oil; jojoba oil; milk bush oil; mustard oil; nahor oil; linseed oil; olive oil; palm oil; pongamia oil; radish oil; ramtil oil; rapeseed oil; rice bran oil; safflower oil; salicornia oil; sesame oil; soybean oil; sunflower oil; tall oil and mixtures and derivatives thereof, the epoxidized plant oil polyol is obtained by opening the oxirane ring of the epoxidized plant oil of step (b), wherein, when the plant oil of step (a) is selected from a group, comprising tall oil and mixtures and derivatives thereof, the epoxidized plant oil polyol is obtained by oxirane ring-opening of the epoxidized plant oil of step (b), and subsequent carboxyl group esterification with alcohols,
wherein the oxirane ring-opening can take place both in the presence and absence of a ring-opening catalyst,
wherein the ring-opening catalyst is selected from boronic acids; hydrobromic acid; hydrochloric acid; perchloric acid; perchloric acid metal salts such as lithium perchlorate; phosphoric acid; phosphorous acid; sulfonic acids such as para-toluene sulfonic acid methanesulfonic acid and trifluoromethane sulfonic acid; sulfuric acid; tetrafluoroboric acid; tetrafluoroboric acid metal salts such as copper tetrafluoroborate, and iron tetrafluoroborate; triflic acid and ion exchange resins in the protic form,
wherein the concentration of the ring-opening catalyst is in a range of 0.01 wt%. to about 1 wt%., preferably 0.05-0.5 wt%,
wherein the epoxidized plant oil is heated up at about 80 °C to 250 °C, preferably about 180 °C, for about 5 to 10 hours,
wherein the alcohol is selected from 2-ethyl-1-hexanol; 2-methyl-2-butanol; ethanol; isobutanol; isopropanol; methanol; n-butanol; n-propanol; propanol; 1,2-cyclohexanediol; 1,3-butanediol; 1,3-propanediol; 1,4-butanediol; 1,5-pentanediol; 1,6-hexanediol; 1,12-dodecanediol; 2,3-butanediol; 2-methyl-1,3-propandiol; 3-methyl-1,5-pentanediol; diethanolamine; ethylene glycol; hexanediol; pentanediol; propylene glycol; triethylene glycol; tripropylene glycol; erythritol; glycerol; pentaerythritol; triethanolamine; trimethylolethane; trimethylolpropane and mixtures thereof,
d) obtaining of Michael donor of Formula (II) and Michael acceptor of Formula (III): 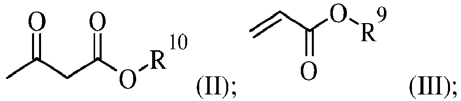
wherein R¹⁰ is plant oil based moiety selected from the group comprising hydrogen; alkyl; alkenyl; oxirane group; hydroxyl -OH; ester -C(=O)-O-; -C=C-; -C=C-; carboxyl group -C(=O)-OH; -C-O-C-; -C=C; acetoacetate -O-C(=O)-C-C(=O)-C, β-ketoester group -O-C(=O)-C-C(=O)-C-; nitrogen-containing group;
wherein R⁹ is plant oil based moiety selected from the group comprising hydrogen or organic radicals such as linear, branched, or cyclic alkyl; alkenyl; oxirane group; hydroxyl -OH; ester -C(=O)-O-; -C=C-; -C=C-; carboxyl group C(=O)-OH; -C-O-C-; -C=C; acrylic -O-C(=O)-C=C; methacrylic -O-C(=O)-C(=C)-C; thio analogs thereof, nitrogen-containing groups, or combinations thereof, including derivatives and substituted versions thereof;
wherein the Michael donor is obtained by subjecting the epoxidized plant oil polyol of step (c) to transesterification, wherein the transesterification is performed using an acetoacetylating reagent in a temperature in a range of 100 to 140 °C, preferably about 120 °C, for 3 to 8 hours, preferably 4 hours,
wherein, when the plant oil of step (a) is selected from a group, comprising coconut oil; canola oil; castor oil; coconut oil; colza oil; copaiba oil; corn oil; cottonseed oil; false flax oil; hemp oil; jatropha oil; jojoba oil; milk bush oil; mustard oil; nahor oil; linseed oil; olive oil; palm oil; pongamia oil; radish oil; ramtil oil; rapeseed oil; rice bran oil; safflower oil; salicornia oil; sesame oil; soybean oil; sunflower oil; tall oil and mixtures and derivatives thereof, the Michael acceptor is obtained by opening an oxirane ring of the epoxidized plant oil of step (b) by acrylating the epoxidized plant oil of step (b) by acrylic group containing acid or an anhydride in the presence or absence of free radical inhibitors at a temperature in a range from 60 to 130 °C, preferably from 100 to 110 °C for 1 to 24 hours, preferably 6 to 8 hours, then washing with a distilled water having a temperature in a range of 40 to 80 °C, and drying;
wherein, when the plant oil of step (a) is selected from a group, comprising tall oil and mixtures and derivatives thereof, the Michael acceptor is obtained by acrylation of the epoxidized plant oil polyol of step (c) with acryloyl chloride or methacryloyl chloride in triethanolamine and ethyl acetate solution in the presence or absence of free radical inhibitors at a temperature in a range from -5 to 5 °C and then stirring the resulting mixture at a temperature in a range from 10 to 30 °C for 12 to 24 hours;
e) mixing of the Michael donor with the Michael acceptor obtained in step (d) in the presence of a catalyst, adding a surfactant and a blowing agent, resulting in a reaction mixture,
wherein a ratio of the Michael acceptor to the Michael donor is in a range of 1:1 to 3:1, preferably 1.5:1 to 2.5:1, more preferably 1.9:1 to 2.1:1,
wherein a concentration of the catalyst is in a range of 0.05 wt% to 10 wt% based upon the total weight of the Michael donor and the Michael acceptor,
wherein a concentration of the surfactant is in a range of 0.1 wt% to 10 wt% based upon the total weight of the Michael donor and the Michael acceptor,
wherein a concentration of the blowing agent is in a range of 0.5 wt% to 50 wt% based upon the total weight of the Michael donor and the Michael acceptor;
f) heating up the reaction mixture for foam forming up to a temperature from 0 °C to 80°C, preferably from 25 °C to 55 °C;
g) mixing of the reaction mixture for 1 second to 300 seconds, preferably for 3 seconds to 120 seconds, more preferably for 5 seconds to 60 seconds,
in the result of steps d), e) and f) the blowing agent is evaporated, forming the polymeric foam material.

2. The method according to claim 1 **characterized in that** the catalyst is selected from the group comprising aliphatic amines; alkanolamines; aromatic amines; amidine; imine; imidazoles; metal salts; nitrogen; 1-(dimethylamino)-2-propanol; 1,1,3,3-tetramethylguanidine; 1,2-dimethylimidazole; 1,4-diazabicyclo[2.2.2]octane; 1,5,7-triazabicyclo[4.4.0]dec-5-ene; 1,5-diazabicyclo[4.3.0]non-5-ene; 1,8-bis(tetramethylguanidino)naphthalene; 1,8-diazabicyclo[5.4.0]undec-7-ene; 1-[bis[3-(dimethylamino)propyl]amino-2-propanol; 1-[N,N-bis(2-hydroxyethyl)amino]-2-propanol; 1-azabicyclo[3.3.0]octane; 1-ethylpiperidine; 2-((dimethylamino)ethyl)methylaminopropanol; 2-(diethylamino)ethanol; 2-(diisopropylamino)ethanol; 2-(dimethylamino)ethyl methacrylate; 2-[2-(dimethylamino)ethoxy]ethanol; 2-ethyl-4-methyl-1H-imiazole; 2-tert-butyl-1,1,3,3-tetramethylguanidine; 4-butanediamine; 7-methyl-1,5,7-triazabicyclo[4,4,0]dec-5-ene; bis(3-aminopropyl)amine; bis(N,N-dimethyl-3-aminopropyl)amine; N-(2-aminopropyl)imidazole; N-(2-hydroxypropyl)-2-methylprop-2-enamide; N-(2-hydroxypropyl)imidazole; N-(3-aminopropyl)-2-pyrrolidinone; N,N,N',N'-tetramethylhexanediamine; N,N,N',N'-tetraacetylethylenediamine; N,N,N',N'-tetramethyl-1,3-propanediamine; N,N,N',N'-tetramethyl-1,4-butanediamine; N,N,N',N'-tetramethyl-1,6-hexanediamine; N,N,N',N'-tetramethyl-2-butene-1,4-diamine; N,N,N',N'-tetramethyldiaminomethane; N,N,N'-trimethyl-N'-hydroxyethyl bis(aminoethyl) ether; N,N-bis(3-dimethylaminopropyl)N-isopropanolamine; N,N-dimefhylpiperazine; N,N-dimethylaminopropylamine; N,N-dimethylaminopropyl-N'-methylethanolamine; N,N-dimethylefhanolamine; N,N-dimethyl-N'-ethylethylenediamine; N,N-dimorpholinodiethylether; N'-butyl-N',N'-dicyclohexylguanidine; N-ethylmorpholine; N-methylimidazole; N-methylmorpholine; potassium hydroxide; potassium phenoxide; potassium phosphate; sodium hydroxide; sodium phenoxide; tertraethylammonium hydroxide; tetramethylamino bis (propylamine); tetramethylammonium hydroxide; tetramethylguanidine; triazabcyclodecene; triethanolamine; trimethylamine; triethylenediamine; trimethylamine; tris(dimethylamino propyl)amine; tris(dimethylaminopropyl)amine; tris-isopropanolamine; N,N,N',N'-tetramethyl-1,4-butanediamine; N,N,N',N'-tetramethylguanidine; N,N-dimethylaminopropyldipropanolamine; N,N-dimethylbenzylamine; N,N-dimethylethanolamine; N,N-dimorpholinodiethyl ether and mixtures thereof, preferably nitrogen.

3. The method according to claim 1 or 2, **characterized in that** the blowing agent is selected from the group comprising hydrocarbons; cyclopentane; isopentane; n-pentane; hydrofluorocarbons; hydrochlorofluorocarbons; hydrofluorocarbons; chlorofluorocarbons; fluorocarbons; chlorocarbons; esters; aliphatic alcohols; carbon dioxide;air; argon; nitrogen; 1,1-difluoroethane; 1,1,1,2,3,4,4,5,5,5-decafluoropentane; 1,1,1,3,3-pentafluorobutane; 1,1,1,3,3-pentafluoropropane; 1,1,2,2,3-pentafluoropropane; 1,1,2,2-tetrafluoroethane; 1,1,4,4,4-hexafluoro-2-butene; 1,1-dichloro-1-fluoroethane; 1,2-dichloro-1,1,2,2-tetrafluoroethane; 1,2-dichloropropane; 1,2-dichlorotetrafluoroethane; 1-chloro-1,1-difluoroethane; 2,2-dichloro-1,1,1-trifluoroethane; cis-1,1,1,4,4,4-hexafluoro-2-butene; cyclopentane; formaldehyde dimethylacetal; isobutane; isopentane; methylbutane; methylformate; n-butane; n-pentane; pentane; perfluoromethane; propane; trichloromonofluoromethane; trichloromonofluoromethane; trichlorotrifluoroethane; and mixtures thereof.

4. The method according to any of claims 1 to 3, **characterized in that** the surfactant is selected from the group comprising alkoxylated polysiloxane; ethoxylated fatty acid; salts of fatty acid; ethoxylated fatty alcohol; salt of sulfonated fatty alcohol; fatty acid ester of sorbitan; and fatty acid ester sorbitan ethoxylate; silicon glycol copolymer and mixtures thereof.

5. The method according to any of claims 1 to 4, **characterized in that** the peroxyacid for epoxidation is selected from the group comprising peroxyformic acid, peroxyacetic acid, trifluoroperoxy acetic acid, benzyloxyperoxy formic acid and mixtures thereof.

6. The method according to any of claims 1 to 5, **characterized in that** the acetoacetylating reagent is selected from the group comprising tert-butyl acetoacetate, methyl acetoacetate, ethyl acetoacetate, or isopropyl acetoacetate and mixtures thereof.

7. The method according to any of claims 1 to 6, **characterized in that** free radical inhibitors are selected from the group comprising substances based on phenolic groups such as hydroquinones; hydroquinone; catechols; phenothiazines; phenonthiazine and mixtures thereof.

8. The method according to any of claims 1 to 7, **characterized in that** one or more additives is/are added to the mix of the Michael acceptor with the Michael donor, wherein one or more additives is/are in a range of 0.10 wt% to 20.0 wt% based upon the total weight of the Michael donor and the Michael acceptor.

9. The method according to claim 8, wherein the one or more additives is/are selected from the group comprising antioxidants; antistatic agents; biocides; colorants; cure promoters; dyes; fillers; flame retardants; foam cell nucleators; foaming agents; functionalized fillers; odor inhibitors; odoriferous substances; perfumes; pigment dispersing agents; plasticizers; preservatives; reactive fillers; UV stabilizers and mixtures thereof.
